# EUROPÄISCHE PATENTSCHRIFT

(11) **EP 2 854 951 B1**
(45) Veröffentlichungstag und Bekanntmachung des Hinweises auf die Patenterteilung: **07.09.2016**
(21) Anmeldenummer: 13725966.9
(22) Anmeldetag: 29.05.2013
(51) Int. Cl.: A61K 8/42, A61K 8/44, A61Q 5/02, A61Q 19/10, C11D 3/43, C11D 1/52, C11D 1/94

(54) **ZUSAMMENSETZUNG ENTHALTEND AMINOSÄURETENSIDE, BETAINE UND N-METHYL-N-ACYLGLUCAMINE MIT VERBESSERTER SCHAUMQUALITÄT UND HÖHERER VISKOSITÄT**
COMPOSITION CONTAINING AMINO ACID SURFACTANTS, BETAINES AND N-METHYL-N-ACYLGLUCAMINES AND HAVING IMPROVED FOAM QUALITY AND HIGHER VISCOSITY
COMPOSITION CONTENANT DES TENSIOACTIFS D'ACIDES AMINÉS, DE LA BÉTAÏNE ET DES N-MÉTHYL-N-ACYLGLUCAMINES, DE QUALITÉ MOUSSANTE AMÉLIORÉE ET DE VISCOSITÉ ACCRUE

(30) Priorität: 30.05.2012 DE 102012010657
(43) Veröffentlichungstag der Anmeldung: 08.04.2015
(73) Patentinhaber: Clariant International Ltd., 4132 Muttenz (CH)
(72) Erfinder: KLUG, Peter, 63762 Großostheim (DE); MILDNER, Carina, 65931 Frankfurt am Main (DE)
(74) Vertreter: Holmes, Rosalind
(86) Internationale Anmeldenummer: PCT/EP2013/061076
(87) Internationale Veröffentlichungsnummer: WO 2013/178684

(56) Entgegenhaltungen:
- WO-A1-2012/029514
- US-B2- 8 178 481

## Beschreibung

Die Erfindung betrifft eine Zusammensetzung enthaltend mindestens eine N-Acyl-aminosäuretensid, ein Betaintensid, ein N-Methyl-N-acylglucamin, ein Lösungsmittel und gegebenenfalls ein oder mehrere Additive sowie ein Verfahren zur Herstellung der Zusammensetzung. Ferner betrifft die Erfindung die Verwendung der Zusammensetzung zur Behandlung oder Pflege der Haut oder Haare oder als Shampoo, Gesichtsreiniger, Flüssigreiniger oder Duschbad.

Die Produktion flüssiger Produkte auf dem Kosmetik und Waschmittelsektor nimmt ständig zu. Speziell im Bereich der Körperreinigungsmittel sind es die flüssigen Haarshampoos, Schaumbäder und Duschbäder, die in den letzten Jahren zunehmend an Bedeutung gewonnen haben. Flüssige Geschirrspülmittel und flüssige Feinwaschmittel haben sich ebenfalls einen festen Platz auf dem Markt erobert. Dabei ist es wichtig für die verschiedenen Anwendungen eine entsprechende Viskosität einzustellen, die durch die Zugabe von Verdickungsmitteln erzielt wird. Idealerweise sind nur geringe Mengen an Verdickungsmitteln oder keine Verdickungsmitteln notwendig.

EP 0 285 786 beschreibt Verwendungen von N-Polyhydroxyalkylfettsäureamiden als Verdickungsmittel für flüssige wässrige Tensidsysteme. Diese Aufgabenstellung wurde gelöst durch Verwendung von N-Polyhydroxyalkylfettsäureamiden der allgemeinen Formel I 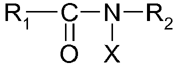
in der R₁ ein gegebenenfalls verzweigter Alkylrest mit 1 bis 17, vorzugsweise 7 bis 17 Kohlenstoffatomen,
R₂ Wasserstoff, ein, gegebenenfalls verzweigter, gegebenenfalls ungesättigter, Alkylrest mit 1 bis 18, vorzugsweise 1 bis 6 Kohlenstoffatomen oder ein Rest 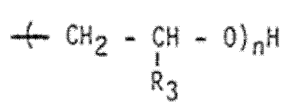 mit n = 0 oder 1 bis 5 und R₃ gleich Wasserstoff oder -CH₃ sein kann und X für einen Polyhydroxyalkylrest mit 4 bis 7 Kohlenstoffatomen steht, der gegebenenfalls mit einem Mono-, Di- oder Oligosaccharidrest glykosidisch verknüpft ist, als Verdickungsmittel für flüssige wässrige Tensidsysteme. Diese Patentschrift beschreibt jedoch nicht die Verdickung von Aminsäuretensiden und verwendet als Verdickungsmittel ein N-Acyl-N-Methylglucamintensid mit einer Alkylkettenverteilung des Kokosöls und damit mit einem geringen Anteil an längerkettigen C₁₆-C₂₀ Glucamiden.

WO 98/56496 betrifft eine Tensidzusammensetzung mit verbesserter Schaumstabilität. Die Tensidzusammensetzung enhält: (a) von ungefähr 1 bis ungefähr 40 Gew.-% eines Zuckertensids; (b) von ungefähr 1 bis ungefähr 40 Gew.-% eines anionischen Tensids; (c) von ungefähr 0,11 bis ungefähr 10 Gew.-% eines Amphoacetats; und (d) Rest Wasser, wobei sich die Gewichtsangaben auf das Gewicht der Zusammensetzung beziehen.

Voraussetzung für eine gute Tensidrezeptur ist eine gute Lagerstabilität. Die Zusammensetzung darf bei Temperaturschwankungen nicht eintrüben oder Absetzungen bilden und sollte eine Viskosität aufweisen, die dem jeweiligen Anwendungszweck angepasst werden kann. Somit ist die Viskosität ein Qualitätskriterium. Der Grad der Viskosität hängt von dem Tensidsystem und dem Elektrolytzusatz ab. Dabei ist es bekannt, dass die aus dem Stand der Technik bekannten Verdickungsmittel in Gegenwart von Paraffinsulfonaten keine ausreichende Viskositätserhöhung aufzeigen (siehe Fette - Seifen - Anstrichmittel 78, 200, (1976)). Dagegen sind Aminosäuretenside wie Acylglycinate, Acylaspartate oder Acylglutamate trotz ihrer guten Hautverträglichkeit in vielen Fällen nicht ausreichend verdickbar und waren bisher deshalb als Primärtensid wegen der hohen nicht ökonomisch. Überraschenderweise wurde gefunden, dass längerkettige, nicht jedoch kürzerkettige, N-Acyl-N-Methylglucamine sich als exzellente Cotenside mit hoher Verdickungsleistung für aminosäuretensidhaltige Zusammensetzungen eignen. Die Aufgabe der vorliegenden Erfindung liegt somit darin, verbesserte Zusammensetzungen bereitzustellen, insbesondere im Hinblick auf eine verbesserte Viskositätseinstellung.

Demgemäß wird eine Zusammensetzung bereitgestellt enthaltend:
(A) mindestens eine N-Acyl-aminosäuretensid als Komponente A,
(B) mindestens ein Betaintensid als Komponente B,
(C) mindestens ein N-Methyl-N-acylglucamin als Komponente C, wobei das N-Methyl-N-acylglucamin einen C₁₆-C₂₀-Acylrest aufweist,
(D) mindestens ein Lösungsmittel als Komponente D, und
(E) gegebenenfalls ein oder mehrere Additive als Komponente E.

Die erfindungsgemäße Zusammensetzung weist vorteilhafterweise eine erhöhte Viskosität auf

Weitere Begriffe für N-Methyl-N-acylglucamin sind N-Methyl-N-1-Desoxysorbitol-Fettsäureamid, N-Acyl-N-methyl-glucamin, Glucamid oder N-Methyl-N-alkylglucamid. Dabei entspricht N-Methyl-N-acylglucamin der Formel (X), wobei R ein organischer Rest ist:

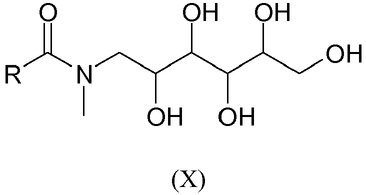

Unter einem N-Acyl-aminosäuretensid wird beispielsweise ein acylierte Aminosäure verstanden.

In Rahmen einer bevorzugten Ausführungsform enthält die Zusammensetzung:
(A) 5 - 15 Gew.-% der Komponente A,
(B) 1 - 10 Gew.-% der Komponente B,
(C) 1 - 10 Gew.-% der Komponente C,
(D) 10 - 93 Gew.-% eines protischen Lösungsmittels als Komponente D, und
(E) 0 - 10 Gew.-% der Komponente E,
wobei die Summe der Komponenten A bis E 100 Gew.-% ergibt.

Bevorzugt besteht die Zusammensetzung aus
(A) 5 - 15 Gew.-% der Komponente A,
(B) 1 - 10 Gew.-% der Komponente B,
(C) 1 - 10 Gew.-% der Komponente C,
(D) 10 - 93 Gew.-% eines protischen Lösungsmittels als Komponente D, und
(E) 0 - 10 Gew.-% der Komponente E,
wobei die Summe der Komponenten A bis E 100 Gew.-% ergibt.

Im Rahmen einer bevorzugten Ausführungsform ist der Aminosäurerest der Komponente A ausgewählt aus der Gruppe bestehend aus proteinogenen Aminosäuren, deren N-alkylierten Derivaten oder Mischungen daraus.

Bevorzugt als Komponente A sind Acylglycinate, Acylalaninate, Acylaspartate, Acylglutamate und Acylsarkosinate, besonders Natrium Cocoyl-Glycinat, Kaliumcocoylglycinat, Natriumlauroylglycinat, Kaliumlauroylglycinat, Natriumcocoylglutamat, Natriumlauroylglutamat, Natriumcocoylaspartat, Natriumlauroylaspartat und Natriumlauroylsarkosinat.

Im Rahmen einer bevorzugten Ausführungsform besteht die Komponente A aus mindestens einer C₈-C₂₂-acylierten Aminosäure, insbesondere deren N-alkylierten Derivaten. Bevorzugt sind die entsprechenden Lauroyl - oder Cocoylderivate der Aminosäuren.

Im Rahmen einer bevorzugten Ausführungsform ist die Komponente A ausgewählt ist aus der Gruppe bestehend aus Acylglycinat, Acylaspartat, Acylglutamat, Acylsarkosinat oder Mischungen daraus.

Im Rahmen einer bevorzugten Ausführungsform umfasst die Komponente B mindestens ein Alkylbetain und/ oder mindestens ein Alkylamidobetain.

Beispiele für geeignete Alkylbetaine stellen die Carboxyalkylierungsprodukte von sekundären und insbesondere tertiären Aminen der Formel (III) dar 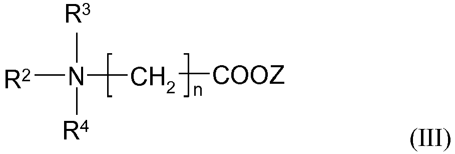 in der R² für Alkyl- und/oder Alkenylreste mit 6 bis 22 Kohlenstoffatomen, R³ für Wasserstoff oder Alkylreste mit 1 bis 4 Kohlenstoffatomen, R⁴ für Wasserstoff oder Alkylreste mit 1 bis 4 Kohlenstoffatomen, n für Zahlen von 1 bis 6 und Z für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C12/14-Kokosalkyldimethylamin, Myristyldimethylamin, Cetyldimethylamin, Stearyldimethylamin, Stearylethylmethylamin, Oleyldimethylamin, C16/18-Talgalkyldimethylamin sowie deren technische Gemische.

Beispiele für geeignete Alkylamoidbetaine stellen Carboxyalkylierungsprodukte von Amidoaminen da. Insbesondere geeignet sind Amidopropylbetaine der Formel (IV), 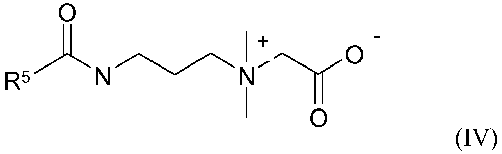 worin R⁵ eine lineare oder verzweigte gesättigte C₇-C₂₁ Alkylgruppe oder eine lineare oder verzweigte ein- oder mehrfach ungesättigte C₇-C₂₁ Alkenylgruppe ist.

Bevorzugten Betaintenside sind Amidopropylbetaine wie Cocoamidopropylbetain (R⁵CO ist der Fettsäurerest des Kocosöls, Kettenlänge C₅-C₁₈) und Alkylbetaine wie Coco-Betain (R² ist der Alkylrest des Kocosöls, Kettenlänge C₈-C₁₈) oder Laurylbetain (R² ist ein Alkylrest der Kettenlänge C₁₂ und C₁₄).

Im Rahmen einer bevorzugten Ausführungsform besteht die Komponente C aus einer Mischung aus N-Methyl-N-acylglucaminen, wobei mindestens 80 Gew.-% der N-Methyl-N-acylglucamine einen gesättigten oder ungesättigten C₁₆- oder C₁₈-Acylrest aufweisen. Beispielsweise können in der Mischung auch N-Methyl-N-acylglucamine vorliegen, die einen C₁₄-Acylrest aufweisen, wobei aber mindestens 80 Gew.-% der N-Methyl-N-acylglucamine einen gesättigten oder ungesättigten C₁₆- oder C₁₈-Acylrest aufweisen. Bevorzugt besteht die Komponente C aus einer Mischung aus N-Methyl-N-acylglucaminen, wobei mindestens 90 Gew.-% der N-Methyl-N-acylglucamine einen gesättigten oder ungesättigten C₁₆- oder C₁₈-Acylrest aufweisen.

Unter einem Lösungsmittel im Rahmen der vorliegenden Erfindung versteht man vorzugsweise protische Lösungsmittel wie Wasser, C₁-C₈-Alkohole, insbesondere C₁-C₆-Alkohole, Ethylenglykol, Diethylenglykol, Triethylenglykol oder Mischungen davon, wobei insbesondere Wasser und/oder Ethanol oder Wasser und/oder Methanol bevorzugt sind. Von den C₁-C₆-Alkoholen sind Methanol, Ethanol, Isopropanol, n-Butanol oder sek.-Butanol bevorzugt.

Im Rahmen einer bevorzugten Ausführungsform ist das Lösungsmittel der Komponente D Wasser oder ein Gemisch aus Wasser und Propylenglykol.

Im Rahmen einer bevorzugten Ausführungsform ist die Zusammensetzung frei von Alkylsulfaten und/oder Alkylethersulfaten. Dabei bedeutet frei, dass die Zusammensetzung weniger als 3 Gew.-%, bezogen auf die Gesamtmenge der Zusammensetzung, bevorzugt weniger als 0,5 Gew.-%, und insbesondere keine Alkylsulfate und/oder Alkylethersulfate beinhaltet.

Im Rahmen einer bevorzugten Ausführungsform beträgt die Summe der Komponenten A, B, und C von 7 bis 20 Gew.-%, bevorzugt von 8 bis 18 Gew.-%, und insbesondere von 10 bis 15 Gew.-%.

Im Rahmen einer bevorzugten Ausführungsform sind die Additive aus der Gruppe bestehend aus Konservierungsmitteln, Duftstoffen, Farbstoffen, weiteren Tensiden, Wasser, Ölkörpern, kationischen Polymeren, Filmbildnern, Verdickungs- und Gelierungsmitteln, Überfettungsmitteln, antimikrobiellen und biogenen Wirkstoffen, feuchtigkeitsspendenen Mitteln, Stabilisatoren, Säuren, Laugen, Wirkverstärkern und Mischungen daraus ausgewählt, bevorzugt in Mengen von 0,1 bis 10,0 Gew.-%, besonders bevorzugt von 0,5 bis 8,0 Gew.-% und insbesondere von 1,0 bis 5,0 Gew.-%.

Als Konservierungsmittel eignen sich in betreffenden Annex der europäischen Kosmetikgesetzgebung gelisteten Konservierungsmittel, beispielsweise Phenoxyethanol, Benzylalkohol, Parabene, Benzoesäure und Sorbinsäure, besonders gut geeignet ist beispielsweise 1,3-Bis(hydroxymethyl)-5,5-dimethylimidazolidine-2,4-dione (Nipaguard^{®} DMDMH), Pirocton Olamin, Methylisothiazolinon oder Mischungen daraus, bevorzugt Pirocton Olamin und/oder Methylisothiazolinon.

Als Duft- bzw. Parfümstoffe oder Öle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die Ionone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z. B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die gewünschte Viskosität der Zusammensetzungen kann durch Zugabe von Verdickern und Gelierungsmittel eingestellt (erhöht oder erniedrigt) werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox, Iodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol, 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen können des Weiteren biogene Wirkstoffe ausgewählt aus Pflanzenextrakten, wie beispielsweise Aloe Vera, sowie Lokalanästhetika, Antibiotika, Antiphlogistika, Antiallergika, Corticosteroide, Sebostatika, Bisabolol®, Allantoin®, Phytantriol®, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Als Stabilisatoren können Metallsalze von Fettsäuren, wie z. B. Magnesium-, Aluminium- und/oder Zinkstearat eingesetzt werden.

Als feuchtigkeitsspendende Substanz stehen beispielsweise Isopropylpalmitat, Glycerin und/oder Sorbitol zu Verfügung. Besonders bevorzugt ist Sorbitol.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol, Polyvinylpyrrolidoncopolymeren wie PVP/Hexandecene oder PVP/Eicosene Copolymer, beispielsweise Vinylpyrrolidon/Vinylacetatcopolymer, wasserlöslichen Acrylsäurepolymeren/Copolymeren bzw. deren Estern oder Salzen, beispielsweise Partialestercopolymere der Acryl/Methacrylsäure und Polyethylenglykolethern von Fettalkoholen, wie Acrylat/Steareth-20-Methacrylat Copolymer, wasserlöslicher Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich, sowie polymeren Aminoxiden, beispielsweise unter den Handelsnamen Diaformer Z-711, 712, 731, 751 erhältliche Vertreter.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden.

Als Säuren oder Laugen zur pH-Wert Einstellung werden vorzugsweise Mineralsäuren, insbesondere HCl, anorganische Basen, insbesondere NaOH oder KOH, oder organische Säuren, insbesondere Milchsäure, verwendet.

Als Wirkverstärker kann bevorzugt Sorbitancaprylat verwendet werden.

Im Rahmen einer bevorzugten Ausführungsform handelt es sich bei der Zusammensetzung um eine kosmetische, dermatologische oder pharmazeutische Zusammensetzung.

Ein weiterer Gegenstand der Erfindung ist ein Verfahren zur Herstellung der erfindungssemäßen Zusammensetzung, umfassend die Schritte:
a) Mischen der Komponente E mit D zu einer Lösung,
b) Zugabe der Komponente A zu der Lösung aus a)
c) Mischen von Komponente D mit gegebenenfalls einer weiteren Komponente E, bevorzugt ein Befeuchtungsmittel, und anschließende Zugabe zu der Lösung aus Schritt b),
d) Zugabe von Komponente B und C, insbesondere nacheinander, zu der Lösung aus c) und
e) Einstellen der Zusammensetzung auf einen pH-Wert von 6,8 bis 7,5.

In einer weiteren Variante wird die erfindungsgemäße Zusammensetzung hergestellt, indem
(A) 5 - 15 Gew.-% der Komponente A,
(B) 1 - 10 Gew.-% der Komponente B,
(C) 1 - 10 Gew.-% der Komponente C,
(D) 10 - 93 Gew.-% eines protischen Lösungsmittels als Komponente D und
(E) 0 - 10 Gew.-% der Komponente E,
wobei die Summe der Komponenten A bis E 100 Gew.-% ergibt,

### verwendet werden.

Ein weiterer Gegenstand der Erfindung ist die Verwendung der erfindungsgemäßen Zusammensetzung als Shampoo, Gesichtsreiniger, Flüssigreiniger oder Duschbad.

Ein weiterer Gegenstand der Erfindung ist die Verwendung der erfindungsgemäßen Zusammensetzung zur Behandlung oder Pflege der Haut.

Ein weiterer Gegenstand der Erfindung ist die Verwendung der erfindungsgemäßen Zusammensetzung zur Behandlung oder Pflege der Haare.

Die Erfindung wird durch die nachstehenden Beispiele näher erläutert.

### Herstellbeispiel H1 und H2, Beispiel 1 und Vergleichsbeispiele 1 und 2

Die im Folgenden beschriebene N-Acyl-N-methyl-glucamine wurden nach EP 0 550 637 aus den korrespondierenden Fettsäuremethylestern und N-Methylglucamin in Gegenwart von 1,2 Propylenglykol als Lösemittel hergestellt und als Feststoff bestehend aus Aktivsubstanz und 1,2 Propylenglykol erhalten.

**Tabelle 1**

| Herstellbeispiel | Methylester | Aktivsubstanz (%) | 1,2 Propylenglykol (%) | Schmelzpunkt |
|---|---|---|---|---|
| H1 | C12/14 | 90 | 10 | 85 |
| H2 | C 16/18 | 80 | 20 | 68 |
| H3 | C16/18' | 80 | 20 | 50 |

| | | | | |
|---|---|---|---|---|
| C12/14 bedeutet, dass der Methylester aus einem Gemisch aus Laurinsäuremethylester ( C₁₂-Acylrest) und Myristinsäuremethylester (C₁₄-Acylrest) besteht (Verhältnis 75 : 25). C16/18 bedeutet, dass der Methylester aus einem Gemisch aus Palmitinsäuremethylester ( C₁₆-Acylrest) und Stearinsäuremethylester (C₁₈-Acylrest) besteht (Verhältnis 30 : 70). C16/18' bedeutet, dass der Methylester aus einem Gemisch aus Palmitinsäuremethylester, Stearinsäuremethylester, Ölsäuremethylester und Linolsäuremethylester (Verhältnis 30 : 10 :50 : 10) besteht. | | | | |

Die Viskositäten werden mit einem Brookfield-Viskosimeter Model DV II, den Spindeln aus dem Spindelset RV bei 20 Umdrehungen / Minute und 20 °C gemessen. Es werden die Spindeln 1 bis 7 aus dem Spindelset RV verwendet. Unter diesen Messbedingungen wird Spindel 1 für Viskositäten von maximal 500 mPa · s, Spindel 2 für Viskositäten von maximal 1 000 mPa · s, Spindel 3 für Viskositäten von maximal 5 000 mPa · s, Spindel 4 für Viskositäten von maximal 10 000 mPa · s, Spindel 5 für Viskositäten von maximal 20 000 mPa · s, Spindel 6 für Viskositäten von maximal 50 000 mPa · s und Spindel 7 für Viskositäten von maximal 200 000 mPa · s gewählt.

In der folgenden Testformulierung wurde das N-Acyl-Methylglucamin nach Herstellbeispiel H2 und H3 im Vergleich zum Herstellbeispiel H1 und im Vergleich zu Alkylpolyglucosiden geprüft.

### Herstellvorschrift

- I: Mischen der Komponenten E (Octopirox^{®} (Clariant)) und D (Wasser).
- II: Zugabe von Komponente A zu I und anschließendes Rühren, bis die Lösung klar ist.
- III: Mischen der Komponenten D (Wasser) und E (Sorbitol) und anschließendes Rühren, bis die Lösung klar ist.
- IV: Zugabe von III zu II und Homogenisieren.
- V: Aufeinanderfolgende Zugabe der Komponenten B (Genagen^{®} KB (Clariant)), C (Zuckertensid) und E (Velsan^{®} SC (Clariant)) zu IV und anschließendes Rühren, bis die Lösung klar ist.
- VI: Einstellen des pH-Werts mittels Komponente E (Milchsäure) auf einen Wert von 7.0 - 7.2.
- VII: Zugabe von Komponente E (SkyBio^{®} M500) zu VII, Homogenisieren.

Die Zusammensetzung und die Herstellung sind in Tabelle 2 zusammengefasst.

**Tabelle 2: Mildes Haarshampoo**

| **Schritt** | **Komponente** | **Bezeichnung** | **Eigenschaft** | |
|---|---|---|---|---|
| | | | | |
| I | E | Octopirox^{®} (Clariant) Pirocton Olamin | Konservierungsmittel | 0.10% |
| | D | Wasser | Lösungsmittel | 10.00% |
| II | A | Hostapon SG^{®} (Clariant) (25 % Lösung in Wasser, berechnet auf Aktivgehalt) Natrium Cocyl Glycinat | Surfactant (Tensid) | 7,50 % |
| III | D | Wasser | Lösungsmittel | ad 100% |
| | E | Sorbitol | Befeuchtungsmittel | 1.00% |
| IV | B | Genagen^{®} KB (Clariant) (30 % Lösung in Wasser, berechnet auf Aktivgehalt) Coco-Betaine | Co-Surfactant (Tensid) | 4,50% |
| | C | Zuckertensid | Co-Surfactant (Tensid) | 4,80 % |
| | E | Velsan^{®} SC (Clariant) Sorbitan Caprylat | Wirkverstärker | 1.00% |
| V | E | Milchsäure, 25% | Neutralisator | bis pH = 7 |
| VI | E | SkyBio^{®} M500 Methylisothiazolinon | Konservierungsmittel | 0.02% |

**Tabelle 3**

| Beispiel | Zuckertensid | Viskosität bei 20 °C (mPas) |
|---|---|---|
| Beispiel 1 | Herstellbeispiel H2 | 27900 |
| Beispiel 2 | Herstellbeispiel H3 | 9500 |
| Vergleichsbeispiel 1 | Herstellbeispiel H1 | 1710 |
| Vergleichsbeispiel 2 | Plantacare 818 (Coco-Glucosid) | 3500 |

Die Einsatzmenge Zuckertensid von 4,8 % bezieht sich auf den Aktivgehalt des Tensids, d. h. die Einsatzmenge Coco-Glucosid (Plantacare 818 /BASF SE) entspricht 9,23 % als Handelsprodukt. Die N-Acyl-N-methylglucamine wurden anhand des Aktivgehalts aus Tabelle 1 eingesetzt.

Wie aus den Beispielen 1 und 2 hervorgeht, zeigen C16/18 N-Acyl-N-methylglucamine eine hervorragende Verdickungsleistung sowohl gegenüber kürzerkettigen N-Acyl-N-methylglucaminen (Vergleichsbeispiel 1) als auch gegenüber Alkylpolyglucosiden (Vergleichsbeispiel 2).

Darüber hinaus sind die Formulierungen auch bei niedrigen Temperaturen viskositätsstabil.

## Patentansprüche

1. Zusammensetzung enthaltend:
(A) mindestens eine N-Acyl-aminosäuretensid als Komponente A,
(B) mindestens ein Betaintensid als Komponente B,
(C) mindestens ein N-Methyl-N-acylglucamin als Komponente C, wobei das N-Methyl-N-acylglucamin einen C₁₆-C₂₀-Acylrest aufweist,
(D) mindestens ein Lösungsmittel als Komponente D und
(E) gegebenenfalls ein oder mehrere Additive als Komponente E.

2. Zusammensetzung nach Anspruch 1, enthaltend:
(A) 5 - 15 Gew.-% der Komponente A,
(B) 1 - 10 Gew.-% der Komponente B,
(C) 1 - 10 Gew.-% der Komponente C,
(D) 10 - 93 Gew.-% eines protischen Lösungsmittels als Komponente D und
(E) 0 - 10 Gew.-% der Komponente E,
wobei die Summe der Komponenten A bis E 100 Gew.-% ergibt.

3. Zusammensetzung nach einem der Ansprüche 1 oder 2, **dadurch gekennzeichnet, dass** der Aminosäurerest der Komponente A ausgewählt ist aus der Gruppe bestehend aus proteinogenen Aminosäuren, deren N-alkylierten Derivaten und Mischungen daraus.

4. Zusammensetzung nach einem der Ansprüche 1 bis 3, **dadurch gekennzeichnet, dass** die Komponente A aus mindestens einer C₈-C₂₂-acylierten Aminosäure besteht.

5. Zusammensetzung nach einem der Ansprüche 1 bis 4, **dadurch gekennzeichnet dass** die Komponente A ausgewählt ist aus der Gruppe bestehend aus Acylglycinat, Acylaspartat, Acylglutamat, Acylsarkosinat oder Mischungen daraus.

6. Zusammensetzung nach einem der Ansprüche 1 bis 5, **dadurch gekennzeichnet, dass** die Komponente B mindestens ein Alkylbetain und/ oder mindestens ein Alkylamidobetain umfasst.

7. Zusammensetzung nach einem der Ansprüche 1 bis 6, **dadurch gekennzeichnet, dass** die Komponente C aus einer Mischung aus N-Methyl-N-acylglucaminen besteht, wobei mindestens 80 Gew.-% der N-Methyl-N-acylglucamine einen gesättigten oder ungesättigten C₁₆- oder C₁₈-Acylrest aufweisen.

8. Zusammensetzung nach einem der Ansprüche 1 oder 7, **dadurch gekennzeichnet, dass** das Lösungsmittel der Komponente D Wasser oder ein Gemisch aus Wasser und Propylenglykol ist.

9. Zusammensetzung nach einem der Ansprüche 1 bis 8, **dadurch gekennzeichnet, dass** die Zusammensetzung frei von Alkylsulfaten und/oder Alkylethersulfaten ist.

10. Zusammensetzung nach einem der Ansprüche 1 bis 9, **dadurch gekennzeichnet, dass** die Summe der Komponenten A, B, und C von 7 bis 20 Gew.-% beträgt.

11. Zusammensetzung nach einem der Ansprüche 1 bis 10, **dadurch gekennzeichnet, dass** die Additive aus der Gruppe bestehend aus Konservierungsmitteln, Duftstoffen, Farbstoffen, weiteren Tensiden, Wasser, Ölkörpern, kationische Polymeren, Filmbildnern, Verdickungs- und Gelierungsmitteln, Überfettungsmitteln, antimikrobiellen und biogenen Wirkstoffen, feuchtigkeitsspendenen Mitteln, Stabilisatoren, Säuren, Laugen, Wirkverstärkern und Mischungen daraus ausgewählt sind.

12. Zusammensetzung nach einem der Ansprüche 1 bis 11, **dadurch gekennzeichnet, dass** es sich bei der Zusammensetzung um eine kosmetische, dermatologische oder pharmazeutische Zusammensetzung handelt.

13. Verfahren zur Herstellung der Zusammensetzung nach einem der Ansprüche 1 bis 12, umfassend die Schritte:
a) Mischen der Komponente E mit D zu einer Lösung,
b) Zugabe der Komponente A zu der Lösung aus a)
c) Mischen von Komponente D mit gegebenenfalls einer weiteren Komponente E und anschließende Zugabe zu der Lösung aus Schritt b),
d) Zugabe von Komponente B und C zu der Lösung aus c) und
e) Einstellen der Zusammensetzung auf einen pH-Wert von 6,8 bis 7,5.

14. Verwendung der Zusammensetzung nach einem der Ansprüche 1 bis 12 als Shampoo, Gesichtsreiniger, Flüssigreiniger oder Duschbad.

15. Verwendung der Zusammensetzung nach einem der Ansprüche 1 bis 12 zur Behandlung oder Pflege der Haut.

## Claims

1. A composition comprising:
(A) at least one N-acylamino acid surfactant as component A,
(B) at least one betaine surfactant as component B,
(C) at least one N-methyl-N-acylglucamine as component C, wherein the N-methyl-N-acylglucamine has a C₁₆-C₂₀-acyl radical,
(D) at least one solvent as component D, and
(E) optionally one or more additives as component E.

2. The composition as claimed in claim 1, comprising:
(A) from 5 to 15% by weight of component A,
(B) from 1 to 10% by weight of component B,
(C) from 1 to 10% by weight of component C,
(D) from 10 to 93% by weight of a protic solvent as component D, and
(E) from 0 to 10% by weight of component E,
wherein the sum of components A to E is 100% by weight.

3. The composition as claimed in either claim 1 or claim 2, wherein the amino acid radical of component A is selected from the group consisting of proteinogenic amino acids, their N-alkylated derivatives and mixtures thereof.

4. The composition as claimed in one of claims 1 to 3, wherein component A consists of at least one C₈-C₂₂-acylated amino acid.

5. The composition as claimed in one of claims 1 to 4, wherein component A is selected from the group consisting of acyl glycinate, acyl aspartate, acyl glutamate, acyl sarcosinate or mixtures thereof.

6. The composition as claimed in one of claims 1 to 5, wherein component B comprises at least one alkyl betaine and/or at least one alkylamido betaine.

7. The composition as claimed in one of claims 1 to 6, wherein component C consists of a mixture of N-methyl-N-acylglucamines, wherein at least 80% by weight of the N-methyl-N-acylglucamines have a saturated or unsaturated C₁₆- or C₁₈-acyl radical.

8. The composition as claimed in one of claims 1 to 7, wherein the solvent of component D is water or a mixture of water and propylene glycol.

9. The composition as claimed in one of claims 1 to 8, wherein the composition is free of alkyl sulfates and/or alkyl ether sulfates.

10. The composition as claimed in one of claims 1 to 9, wherein the sum of components A, B and C is from 7 to 20% by weight.

11. The composition as claimed in one of claims 1 to 10, wherein the additives are selected from the group consisting of preservatives, fragrances, dyes, further surfactants, water, oily substances, cationic polymers, film-forming agents, thickeners and gelling agents, superfatting agents, antimicrobial and biogenic active ingredients, moisture-donating agents, stabilizers, acids, lyes, activity enhancers, and mixtures thereof.

12. The composition as claimed in one of claims 1 to 11, wherein the composition is a cosmetic, dermatological or pharmaceutical composition.

13. A method for preparing the composition as claimed in one of claims 1 to 12, comprising the steps:
a) mixing component E with D to form a solution,
b) adding component A to the solution from a),
c) mixing component D with optionally a further component E and then adding the mixture to the solution from step b),
d) adding components B and C to the solution from c), and
e) adjusting the composition to a pH of from 6.8 to 7.5.

14. The use of the composition as claimed in one of claims 1 to 12 as a shampoo, facial cleanser, liquid cleanser or shower gel.

15. The use of the composition as claimed in one of claims 1 to 12 for the treatment or care of the skin.

## Revendications

1. Composition contenant :
(A) au moins un tensioactif de type acide N-acyl-aminé en tant que composant A,
(B) au moins un tensioactif de type bétaïne en tant que composant B,
(C) au moins une N-méthyl-N-acylglucamine en tant que composant C, la N-méthyl-N-acylglucamine comportant un radical acyle en C₁₆-C₂₀,
(D) au moins un solvant en tant que composant D et
(E) au moins un ou plusieurs additifs en tant que composant E.

2. Composition selon la revendication 1, contenant :
(A) 5 - 15 % en poids du composant A,
(B) 1 - 10 % en poids du composant B,
(C) 1 - 10 % en poids du composant C,
(D) 10 - 93 % en poids d'un solvant protique en tant que composant D et
(E) 0 - 10 % en poids du composant E,
la somme des composants A à E étant égale à 100 % en poids.

3. Composition selon l'une quelconque des revendications 1 et 2, **caractérisée en ce que** le radical acide aminé du composant A est choisi dans le groupe constitué par des acides aminés protéinogènes, leurs dérivés N-alkylés et des mélanges de ceux-ci.

4. Composition selon l'une quelconque des revendications 1 à 3, **caractérisée en ce que** le composant A consiste en au moins un acide aminé acylé avec un groupe acyle en C₈-C₂₂.

5. Composition selon l'une quelconque des revendications 1 à 4, **caractérisée en ce que** le composant A est choisi dans le groupe constitué par un acylglycinate, acylaspartate, acylglutamate ou des mélanges de ceux-ci.

6. Composition selon l'une quelconque des revendications 1 à 5, **caractérisée en ce que** le composant B comprend au moins une alkylbétaïne et/ou au moins une alkylamidobétaïne.

7. Composition selon l'une quelconque des revendications 1 à 6, **caractérisée en ce que** le composant C consiste en un mélange de N-méthyl-N-acylglucamines, au moins 80 % en poids des N-méthyl-N-acylglucamines comportant un radical acyle en C₁₆ ou C₁₈ saturé ou insaturé.

8. Composition selon l'une quelconque des revendications 1 ou 7, **caractérisée en ce que** le solvant D est l'eau ou un mélange d'eau et de propylèneglycol.

9. Composition selon l'une quelconque des revendications 1 à 8, **caractérisée en ce que** la composition est exempte d'alkylsulfates et/ou d'alkyléthersulfates.

10. Composition selon l'une quelconque des revendications 1 à 9, **caractérisée en ce que** la somme des composants A, B et C vaut de 7 à 20 % en poids.

11. Composition selon l'une quelconque des revendications 1 à 10, **caractérisée en ce que** les additifs sont choisis dans le groupe constitué par des conservateurs, des parfums, des colorants, d'autres tensioactifs, l'eau, des corps huileux, des polymères cationiques, des agents filmogènes, des épaississants et gélifiants, des agents de regraissage, des substances actives antimicrobiennes et biogènes, des agents hydratants, des stabilisants, des acides, des solutions alcalines, des activateurs et des mélanges de ceux-ci.

12. Composition selon l'une quelconque des revendications 1 à 11, **caractérisée en ce que** pour ce qui est de la composition il s'agit d'une composition cosmétique, dermatologique ou pharmaceutique.

13. Procédé pour la préparation de la composition selon l'une quelconque des revendications 1 à 12, comprenant les étapes :
a) mélange du composant E avec D pour l'obtention d'une solution,
b) addition du composant A à la solution provenant de a)
c) mélange du composant D avec éventuellement un autre composant E et addition subséquente à la solution provenant de l'étape b),
d) addition des composants B et C à la solution provenant de c) et
e) ajustement de la composition à un pH de 6,8 à 7,5.

14. Utilisation de la composition selon l'une quelconque des revendications 1 à 12, en tant que shampooing, nettoyant pour le visage, produit de nettoyage liquide ou gel douche.

15. Utilisation de la composition selon l'une quelconque des revendications 1 à 12, pour le traitement ou le soin de la peau.
